(19) Europäisches Patentamt
European Patent Office
Office européen des brevets

(11) Numéro de publication: **0 299 846**
**A1**

(12) **DEMANDE DE BREVET EUROPEEN**

(21) Numéro de dépôt: **88401740.1**

(22) Date de dépôt: **05.07.88**

(51) Int. Cl.4: **A 61 K 9/50**
C 08 L 83/04, C 08 K 3/08,
B 01 J 13/02

(30) Priorité: **15.07.87 FR 8709918**

(43) Date de publication de la demande:
**18.01.89 Bulletin 89/03**

(84) Etats contractants désignés:
**AT BE CH DE ES FR GB GR IT LI LU NL SE**

(71) Demandeur: **RHONE-POULENC CHIMIE**
**25, quai Paul Doumer**
**F-92408 Courbevoie Cédex (FR)**

(72) Inventeur: **Leising, Frédéric**
**933 route de Givors Cornevend**
**F-69390 Vernaison (FR)**

**Chauvel, Bernard**
**24, chemin de la Commanderie**
**F-95120 Ermont (FR)**

**Torres, Chislaine**
**22, rue Pauline Marie Garicot**
**F-69005 Lyon (FR)**

(74) Mandataire: **Fabre, Madeleine-France et al**
**RHONE-POULENC INTERSERVICES Service Brevets Chimie 25, quai Paul Doumer**
**F-92408 Courbevoie Cédex (FR)**

(54) **Microsphères magnétisables à base de polysiloxane, leur procédé de préparation et leur application en biologie.**

(57) Microsphères magnétisables, telles quelles ou en dispersions aqueuses, constituées d'une matrice d'au moins un organopolysiloxane portant éventuellement des groupes Si-vinyle ou des motifs réactifs et/ou ionogènes, et encapsulées dans ladite matrice, de particules magnétisables de taille inférieure à 300 Å.

Elles sont préparées par homogénéisation d'une solution dudit organopolysiloxane et d'un fluide magnétique en présence d'eau et d'un tensio-actif, élimination du solvant de l'organopolysiloxane et du liquide vecteur du fluide magnétique et élimination au moins partielle de l'eau.

Elles peuvent être utilisées en biologie.

EP 0 299 846 A1

## Description

### MICROSPHERES MAGNETISABLES A BASE DE POLYSILOXANE, LEUR PROCEDE DE PREPARATION ET LEUR APPLICATION EN BIOLOGIE

La présente invention a pour objet des microsphères magnétisables à base d'organopolysiloxane, se présentant telles quelles ou en dispersion aqueuse, leur procédé de préparation et leur application en biologie.

Selon l'invention, il s'agit de microsphères magnétisables, se présentant telles quelles ou en dispersion aqueuse et constituées :
- d'une matrice à base d'au moins un organopolysiloxane de formule (I)
$R''R'RSiO(SiR_2O)_n(SiRR''O)_mSiRR'R''$ (I)
formule dans laquelle :
. les symboles R sont identiques ou différents et représentent un radical alkyle en $C_1$-$C_3$, phényle ou trifluoro-3,3,3 propyle ;
. les symboles R' représentent un groupement OH ou le symbole R ;
. le symbole R'' représente le symbole R, un radical vinyle ou un radical -R'''-X, où R''' représente un radical organique bivalent et X un groupement réactif et/ou ionogène ;
. au moins 50 % des radicaux représentés par le symbole R sont des radicaux méthyles ;
. les symboles n et m représentent des nombres entiers ou fractionnés ayant une valeur suffisante pour assurer une viscosité du polymère de 20 à 10.000.000 mPas à 25° C, de préférence de l'ordre de 50 à 7.000.000 mPas, et un nombre de motifs réactifs et/ou ionogènes éventuels par molécule d'organopolysiloxane allant de 1 à 1.000, de préférence de l'ordre de 5 à 500 ;
- et, encapsulées dans ladite matrice, de particules magnétisables présentant une taille généralement inférieure à 300 Å et, de préférence, de l'ordre de 80 à 120 Å.

Parmi les motifs diorganosiloxy ne contenant pas de groupements réactifs ou ionogènes, on peut citer ceux de formule suivante :
$(CH_3)_2SiO$ ; $CH_3(CH_2{=}CH_2)SiO$ ; $CH_3(c_2H_5)SiO$ ;
$CH_3(C_6H_5)SiO$ ; $(C_6H_5)_2SiO$ ; $CF_3CH_2CH_2(CH_3)SiO$.

Parmi les motifs triorganosiloxy bloqueurs, on peut citer ceux de formule suivante :
$(CH_3)_3SiO_{0,5}$ ; $(CH_3)_2CH_2{=}CHSiO_{0,5}$ ; $(CH_3)_2C_6H_5SiO_{0,5}$ ;
$CH_3(C_6H_5)_2SiO_{0,5}$ ; $CH_3(CH_2{=}CH)C_6H_5SiO_{0,5}$ ;
$CH_2{=}CH(C_6H_5)SiO_{0,5}$ ; $(C_6H_5)_3SiO_{0,5}$.

Les polyorganosiloxanes bloqués par des radicaux Si-OH ou triorganosiloxy et ne comportant pas de groupements réactifs ou ionogènes sont des produits bien connus ; ils peuvent être préparés selon les procédés décrits dans les brevets français n° 1.134.005, 1.198.749, 1.226.745, 978.058, 1.025.150.

Parmi les radicaux organiques divalents pouvant constituer le symbole R''', on peut citer : les radicaux alkylènes linéaires ou ramifiés en $C_1$-$C_{18}$, éventuellement prolongés par 1 à 5 groupes bivalents éthylène amine, par 1 à 50 groupes oxyde d'alkylène en $C_1$-$C_3$, ou par un groupe $-O-CH_2-\underset{OH}{\underset{|}{CH}}-CH_2-$ ; les radicaux polyoxyalkylènes contenant de 1 à 50 chaînons oxyalkylène en $C_1$-$C_3$.

A titre d'exemple de radicaux bivalents, on peut citer :

$-CH_2-$ ; $-(CH_2)_2-$ ; $-(CH_2)_3-$ ; $-CH_2-\underset{CH_3}{\underset{|}{CH}}-CH_2$ ; $-(CH_2)_{10}-$ ; $-(CH_2)_{12}-$ ;

$-(CH_2)_3NH-CH_2-CH_2-$ ; $-(CH_2)_3-OCH_2-$ ; $-(CH_2)_3-(OCH_2-CH_2)_{29}-$ ;

$-(CH_2)_3-[O-CH_2-CH(CH_3)]_{15}$ ; $-(CH_2)_3OCH_2-\underset{OH}{\underset{|}{CH}}-CH_2-$

Parmi les groupements réactifs ou ionogènes X pouvant constituer le symbole X, on peut citer les groupements : époxy, hydroxy, carboxy, aldéhyde, ester, acétoester, mercapto, mercaptoester, mercaptoalcoxy, amino, alkylamino, dialkylamino, trialkylamino, ammonium quaternaire, aminoalcool, amido, hydrazide, hydrazino, halogénoalkyle en $C_1$-$C_3$, halogénobenzyle, cyano, cyanato,

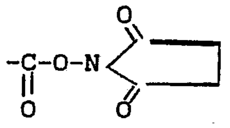 ,

sulfate, sulfonyle.

Les organopolysiloxanes de formule I et portant des groupes réactifs ou ionogènes sont pour la plupart des produits du commerce. Ils peuvent être préparés selon des méthodes bien connues, par exemple :
- par réaction d'addition d'un organohydrogénopolysiloxane sur un composé insaturé $CH_2=CH$-r-X, où le groupement $CH_2=CH$-r- correspond au radical bivalent R''' par ouverture de la double liaison (brevets anglais n° 2.138.185, américain n° 4.558.147, français n° 2.447.942);
- par réaction de condensation d'un organopolyhydroxysiloxane et d'un alcoxysilane ou d'un hydrogénosilane portant un groupement -R'''X (brevet américain n° 4.476.188) ou sur un cétène ;
- par réaction de condensation d'un organohydrogénopolysiloxane et d'un hydroxy ou alcoxysilane portant une fonction -R'''X ;
- par réaction de substitution nucléophile telle que celle d'un organoépoxysiloxane et d'une amine, d'un alcool, d'un thiol, d'un acide ... (brevet anglais n° 2.138.845).

Parmi les matériaux pouvant constituer les particules magnétisables encapsulées dans la matrice de polyorganosiloxane, on peut citer la magnétite, la maghémite, le dioxyde de chrome, les ferrites telles que les ferrites de manganèse, nickel, manganèse-zinc ..., les alliages de cobalt, nickel, gadolinium, samarium-cobalt... Les matériaux préférentiels sont la magnétite et la maghémite.

La quantité de particules magnétisables encapsulées dans la matrice de polyorganosiloxane correspond à environ 0,5 à 50 % en poids desdites particules par rapport au poids de matrice et de préférence de 0,5 à 35 % en poids.

Les microsphères magnétisables faisant l'objet de l'invention peuvent être de taille uniforme ou présenter une granulométrie étalée ; leur diamètre peut être de l'ordre de 0,05 à 3 microns et généralement de l'ordre de 0,2 à 2 microns.

Elles peuvent se présenter telles quelles ou en dispersion dans l'eau ; la quantité de microsphères magnétisables à l'état dispersé dans l'eau peut correspondre à environ 10 à 70 % en poids par rapport au poids total de dispersion et généralement de l'ordre de 15 à 50 % en poids.

La présente invention a également pour objet un procédé de préparation desdites microsphères magnétisables.

Le procédé consiste :

1. à introduire dans un milieu aqueux contenant un tensio-actif, un mélange
   . d'une solution d'au moins un organopolysiloxane de formule I dans un solvant organique de point d'ébullition inférieur à 100°C ou susceptible de former avec l'eau un azéotrope de point d'ébullition inférieur à 100°C et de préférence inférieur à 95°C
   . et d'un fluide magnétique constitué de particules magnétisables de taille généralement inférieure à 300 Å et de préférence de l'ordre de 80 à 120 Å en suspension dans un liquide vecteur organique solvant dudit organopolysiloxane et de point d'ébullition inférieur à 100°C ou susceptible de former avec l'eau un azéotrope de point d'ébullition inférieur à 100°C et de préférence inférieur à 95 °C,
2. à homogénéiser le milieu obtenu,
3. à éliminer par distillation le solvant organique et le liquide vecteur organique,
4. et éventuellement à éliminer l'eau au moins partiellement.

Parmi les solvants de polyorganosiloxane de formule I pouvant être mis en oeuvre, on peut citer le cyclohexane, le chlorure de méthylène, le benzène, l'hexane, l'octane, le toluène, le tétrachlorure de carbone ...

Le solvant est mis en oeuvre afin d'obtenir une viscosité de la solution inférieure à 1.000 mPas à 25°C, de préférence inférieure à 500 mPas . Il est évident qu'un solvant peut ne pas être nécessaire lorsque le poids moléculaire de l'organopolysiloxane est suffisamment bas.

Les fluides magnétiques sont appelés couramment "ferrofluides". Ce sont des suspensions colloïdales extrêmement stables de particules ferro ou ferrimagnétiques de dimension inférieure au micron, dans un liquide vecteur ; ils restent fluides en présence des champs magnétiques externes.

Ce type de matériaux pouvant constituer les particules ferro ou ferrimagnétiques a déjà été mentionné ci-dessus ; les matériaux préférentiels sont la magnétite et la maghémite.

Selon l'invention, le liquide vecteur peut être tout liquide organique solvant du polyorganosiloxane de formule I ou susceptible de former avec l'eau un azéotrope de point d'ébullition inférieur à 100°C, et présentant un point d'ébullition inférieur à 100°C. Tout en étant du même type, le liquide vecteur du fluide magnétique utilisé peut être semblable ou différent du solvant du polyorganosiloxane mis en oeuvre.

Ledit fluide magnétique peut être préparé d'une manière connue, par exemple, selon le procédé décrit dans le brevet américain n° 3.843.540.

Sa préparation fait intervenir un agent dispersant soluble dans l'eau et décomposable thermiquement sous forme non hydrosoluble mais soluble dans le véhicule liquide organique ; en se décomposant, l'agent

dispersant forme un revêtement non hydrosoluble autour de chaque particule magnétisable. Les agents dispersants utiles dans cette préparation peuvent être des acides gras, des amines, des amides ... contenant au moins 12 atomes de carbone, et plus particulièrement les acides gras contenant environ 18 atomes de carbone, tels que les acides oléiques, linoléiques et linoléniques.

La concentration dudit fluide magnétique en particules magnétisables peut être de l'ordre de 20 à 60 % en poids et généralement de l'ordre de 30 à 60 % en poids.

La quantité de fluide magnétique mis en oeuvre pour réaliser le procédé faisant l'objet de l'invention est telle que le poids de particules magnétisables dudit fluide magnétique corresponde à environ 0,5 à 50 % du poids de polyorganosiloxane de formule I, de préférence de 0,5 à 35 %.

Le milieu aqueux dans lequel est introduit le mélange de solution de polyorganosiloxane et de fluide magnétique, peut présenter une concentration en poids de tensio-actif de l'ordre de 0,5 à 15 %, de préférence de l'ordre de 1 à 10 %.

Le tensio-actif contenu dans le milieu aqueux dans lequel est introduit le mélange de solution d'organopolysiloxane et de fluide magnétique peut être tout émulsifiant de type non-ionique, anionique ou cationique hydrosoluble ou susceptible de former des micelles dans l'eau.

Parmi les tensio-actifs non-ioniques, on peut citer les alcools gras polyéthoxylés, les alkylphénols polyéthoxylés, les acides gras polyéthoxylés, les condensats d'oxydes d'éthylène et de propylène, les amides gras polyéthoxylés, les amines grasses polyéthoxylées, les esters d'acides gras, les éthanolamides ...

Parmi les tensio-actifs anioniques, on peut citer les alkylsulfates, alkylsulfonates, alkylarylsulfonates, sulfosuccinates, sulfosuccinates de sodium ...

Parmi les tensio-actifs cationiques, on peut citer les halogénures d'amines grasses, les halogénures, sulfates, méthylsulfates, acétates d'amines grasses éthoxylées, les halogénures d'ammonium quaternaire en $C_{10}$-$C_{18}$ ...

La quantité de milieu aqueux pouvant être mise en oeuvre est telle que la quantité de tensio-actif corresponde à environ 0,5 à 60 % en poids, de préférence de l'ordre de 1 à 50 % en poids par rapport au poids de polyorganosiloxane de formule I.

L'opération d'introduction dans le milieu aqueux du mélange solution de polyorganosiloxane/fluide magnétique est réalisée progressivement sous agitation à température ordinaire (de l'ordre de 15 à 40°C).

L'opération d'homogénéisation est réalisée en une ou plusieurs étapes à une température de l'ordre de 20 à 60°C, à l'aide d'un système d'agitation énergique tel que moulin à colloïdes, pompe à haute pression, agitateur à vibrations, appareil à ultra-sons... jusqu'à obtenir une dispersion de gouttelettes de la phase organique contenant les particules magnétisables, gouttelettes de taille comprise entre environ 0,065 à 3,2 microns et de préférence de 0,35 à 2,2 microns. Lesdites gouttelettes sont constituées de polyorganosiloxane de formule I gonflé de solvant(s) et renfermant les particules magnétisables.

Le ou les solvants du polyorganosiloxane sont ensuite éliminés par distillation sous vide.

On obtient ainsi une dispersion aqueuse de microsphères de dimension pouvant aller de 0,05 à 3 microns, de préférence de 0,2 à 2 microns et constituées d'une matrice à base de polyorganosiloxane de formule I, et, encapsulées dans ladite matrice, de particules magnétisables de dimension inférieure à 300 Å et de préférence de l'ordre de 80 à 120 Å.

Le poids de microsphères magnétisables en dispersion aqueuse peut être ajusté à volonté, soit par élimination partielle de l'eau après aimantation, soit par élimination totale de l'eau après aimantation et ajout d'eau déionisée jusqu'à obtenir un taux d'extrait sec de l'ordre de 10 à 70 % en poids et de préférence de l'ordre de 15 à 50 % en poids.

Si désiré, les microsphères peuvent, bien entendu, être séparées du milieu par simple aimantation.

Les microsphères magnétisables faisant l'objet de l'invention présentent des particularités qui les rendent notamment intéressantes en biologie.

En effet, les microsphères magnétisables de l'invention présentent les avantages suivants :

. elles sont stérilisables à chaud, pendant 2 heures à 122°C,ce qui signifie qu'elles restent actives après stérilisation ;

. les particules magnétisables qu'elles renferment sont enrobées par la matrice de silicone, ce qui évite toute interaction desdites particules avec le milieu réactionnel dans lequel les microsphères sont utilisées ;

. elles sont biotolérantes et non toxiques, ce qui leur permet de ne pas perturber les processus biologiques in vitro et de pouvoir être utilisées in vivo ;

. elles sont magnétisables, ce qui leur permet d'être séparées par simple aimantation du milieu réactionnel dans lequel elles ont été utilisées ; les opérations de lavage sont également de ce fait accélérées.

Lesdites microsphères magnétisables peuvent être utilisées par exemple comme supports actifs :

. d'anticorps ou d'antigènes pour les tests de diagnostiques, les séparations par affinité des composés biologiques ; la fixation des molécules biologiques peut, si nécessaire, être réalisée par des méthodes de couplage bien connues faisant intervenir des agents de couplage (glutaraldéhyde, carbodiimide hydrosoluble), ou bien consistant à activer les fonctions éventuelles du polyorganosiloxane (par exemple par diazotation, par action du bromure de cyanogène, de l'hydrazine ...) et à faire réagir la molécule à fixer, etc...

. de systèmes enzymatiques pour réactions biologiques

. de fixation de cultures cellulaires

. de médicaments ou de substances de révélation pour guider ceux-ci ou celles-ci in vitro ou in vivo vers le point de traitement choisi

. de molécules chimiques permettant une croissance de ces molécules par enchaînement rapide de réactions particulières comme la synthèse peptidique
. de groupements chimiques catalyseurs de réaction
. de groupements chimiques pour la séparation ou l'extraction de métaux ou d'isomères optiques.

Les exemples suivants sont donnés à titre indicatif et ne peuvent être considérés comme une limite du domaine et de l'esprit de l'invention.

Les ferrofluides mis en oeuvre pour réaliser les exemples ont été préparés selon le mode opératoire général suivant :

On dissout successivement dans 31 kg d'eau 11 kg de $FeCl_3,6H_2O$, puis 7,5 kg de $FeSO_4,7H_2O$. La solution obtenue est introduite dans un réacteur contenant 20 kg d'une solution aqueuse à 20 % d'ammoniaque. Le réacteur est porté à 60°C et maintenu à cette température pendant 15 minutes ; on ajoute 2,4 kg d'acide oléique et l'on maintient le milieu sous agitation à 60°C pendant 15 minutes ; le milieu est refroidi à 25°C puis neutralisé par de l'acide chlorhydrique à 38 % jusqu'à obtenir un pH de 5,5.

Après filtration sous vide, le produit est lavé à l'eau, puis à l'acétone et séché. Le produit est repris par un solvant organique (liquide vecteur) puis l'eau résiduelle éliminée par distillation azéotropique.

La quantité de liquide vecteur est telle que la concentration en magnétite formée est de 50 % en poids.

La taille des particules de magnétite, mesurée par microscopie électronique est de l'ordre de 100 Å.

Exemple 1

13 g d'un ferrofluide dont le liquide vecteur est le cyclohexane (ce qui correspond donc à 6,5 g de magnétite) sont introduits dans une solution constituée de 400 g de cyclohexane et de 35 g de polydiméthylsiloxane α, ω dihydroxylé (préparé par polycondensation d'oligomères polydiméthylsiloxanols) de caractéristiques suivantes :

$\overline{Mn}$ = 145.000   $\overline{Mw}$ = 312.000

Viscosité : 1.500.000 mPas à 25°C

Le mélange obtenu est homogénéisé en présence de 800 g d'eau et de 10 g de dodécylbenzènesulfonate de sodium (ce qui correspond à 28,5 % en poids par rapport à l'huile silicone), dans une cuve à ultra-sons (appareil SONIFIER B-30 commercialisé par BRANSON SONIC POWER CO) jusqu'à obtenir un diamètre moyen des microsphères en dispersion de 0,8 micron.

Le cyclohexane est ensuite éliminé à 40°C sous pression réduite (175 mbar). Les dernières traces de cyclohexane sont éliminées par distillation azéotropique avec de l'acide acétique ; la teneur résiduelle en cyclohexane est inférieure à 15 ppm (mesure par chromatographie en phase gazeuse).

On obtient ainsi une dispersion de microsphères dont le diamètre moyen est de 0,65 micron (mesure fait au COULTER NANOSIZER PSM série 17 commercialisé par Coulter Electronics Ltd) et renfermant 18,5 % en poids de particules magnétiques d'une taille de l'ordre de 100 Å.

Les microsphères sont séparées par aimantation, puis redispersées dans de l'eau déionisée jusqu'à obtenir un taux d'extrait sec de 47 %.

Exemple 2

On répète l'exemple 1 en remplaçant le cyclohexane par la même quantité de toluène, à la fois comme liquide vecteur et comme solvant du polymère.

Le diamètre moyen des microsphères magnétisables est de 0,75 micron avant élimination du toluène et de 0,40 micron après élimination des dernières traces de toluène.

Le taux de particules magnétiques dans les microsphères est de 18,5 %.

La dispersion aqueuse est ajustée comme à l'exemple 1 afin d'avoir un extrait sec de 47 %.

Exemple 3

On répète l'exemple 1 en remplaçant le cyclohexane par la même quantité de chlorure de méthylène, à la fois comme liquide vecteur et comme solvant du polymère.

Le diamètre moyen des microsphères magnétisables est de 0,95 micron avant élimination du chlorure de méthylène et de 0,80 micron après élimination des traces de chlorure de méthylène.

Le taux de particules magnétiques dans les microsphères est de 18,5 %.

La dispersion aqueuse est ajustée selon la méthode de l'exemple 1 afin d'obtenir un extrait sec de 30 %.

Exemple 4

L'opération décrite à l'exemple 1 est répétée en utilisant comme homogénéiseur le MANTON-GAULIN (commercialisé par la Société Manton-Gaulin).

Sous une pression de 450 kg/cm² et un premier passage, on obtient une granulométrie moyenne de la dispersion de 0,80 micron ; après un deuxième passage, celle-ci est de 0,65 micron.

Le diamètre moyen des microsphères magnétisables après élimination du cyclohexane est de 0,5 micron ; le taux de particules magnétiques dans les microsphères est de 18,5 % en poids.

La dispersion aqueuse est ajustée selon la méthode de l'exemple 1 afin d'obtenir un extrait sec de 20 %.

Exemple 5

L'opération décrite à l'exemple 1 est répétée en utilisant comme homogénéiseur le SUPRATON (commercialisé par KRUPP TECHNIQUES INDUSTRIELLES).

Après un passage, on obtient une granulométrie moyenne de la dispersion de l'ordre de 1 micron.

Après élimination du cyclohexane, le diamètre moyen des microsphères magnétisables est de 0,9 micron ; le taux de particules magnétiques dans les microsphères est de 18,5 % en poids.

La dispersion aqueuse est ajustée selon la méthode de l'exemple 1 afin d'obtenir un extrait sec de 15 %.

Exemple 6

L'opération décrite à l'exemple 1 est répétée en utilisant comme homogénéiseur l'ULTRA TURRAX IKA WERK (commercialisé par Prolabo). La granulométrie moyenne de la dispersion est de l'ordre de 1,4 micron avant élimination du cyclohexane ; après élimination du cyclohexane, le diamètre moyen des microsphères magnétisables est de 1,2 micron ; le taux de particules magnétisable est de 18,5 % en poids.

Exemple 7

15 g de ferrofluide, dont le liquide vecteur est le cyclohexane (ce qui correspond à 7,5 g de magnétite), sont introduits dans une solution constituée de 400 g de cyclohexane et de 75 g de polydiméthylsiloxane $\alpha$, $\omega$ dihydroxylé de caractéristiques suivantes :

$\overline{Mn}$ = 179.000 $\overline{Mw}$ = 535.000

Viscosité : 6.500.000 mPas à 25°C

Le mélange obtenu est homogénéisé en présence de 800 g d'eau et de 23,5 g (ce qui correspond à 31 % en poids par rapport au polymère) de CEMULSOL ON 10-20 (mélange d'octyl phénoléthoxylé et de nonylphénoléthoxylé commercialisé par la Société Française d'Organo-Synthèse) dans une cuve à ultra-sons (appareil SONIFIER B 30). Après 5 minutes d'homogénéisation, on obtient une dispersion de microsphères magnétisables présentant une granulométrie moyenne de 0,95 micron. Après élimination du cyclohexane, la granulométrie moyenne est de 0,8 micron.

Les microsphères renferment 10 % en poids de particules magnétisables d'une taille de l'ordre de 100 Å.

La dispersion aqueuse est ajustée selon la méthode de l'exemple 1 afin d'obtenir un extrait sec de 15 %.

Exemple 8

20 g d'un ferrofluide dont le liquide vecteur est le cyclohexane (ce qui correspond à 10 g de magnétite) sont introduits dans une solution constituée de 185 g de chlorure de méthylène et de 20 g d'un polydiméthylsiloxane $\alpha,\omega$ dihydroxylé de caractéristiques suivantes :

$\overline{Mn}$ = 145.000 $\overline{Mw}$ = 312.000

Viscosité : 1.500.000 mPas à 25°C

Le mélange obtenu est homogénéisé en présence de 800 g d'eau et de 10 g de laurylsulfate de sodium, ce qui correspond à 50 % en poids par rapport au polymère dans une cuve à ultra-sons (appareil SONIFIER B 30). Après 5 minutes d'homogénéisation, on obtient une dispersion de microsphères magnétisables présentant une granulométrie moyenne de 0,80 micron.

Après élimination du chlorure de méthylène, la granulométrie moyenne est de 0,7 micron.

Les microsphères renferment 50 % en poids en particules magnétisables de taille de l'ordre e 100 Å.

La dispersion aqueuse est ajustée selon la méthode de l'exemple 1 afin d'obtenir un extrait sec de 20 %.

Exemple 9

20 g d'un ferrofluide dont le liquide vecteur est le toluène (ce qui correspond à 10 g de magnétite) sont introduits dans une solution constituée de 500 g de toluène et de 50 g d'un polydiméthylsiloxane $\alpha,\omega$ dihydroxylé de caractéristiques suivantes :

$\overline{Mn}$ = 96.000 $\overline{Mw}$ = 205.000

Viscosité : 2.500.000 mPas à 25°C

Le mélange obtenu est homogénéisé en présence de 800 g d'eau et 3,5 de dodécylbenzènesulfonate de sodium dans une cuve à ultra-sons (SONIFIER B 30). Après 30 minutes d'homogénéisation, on obtient une dispersion de microsphères magnétisables présentant une granulométrie moyenne de 0,55 micron. Après élimination du toluène, la granulométrie moyenne est de 0,45 micron.

Les microsphères renferment 20 % en poids de particules magnétisables de taille de l'ordre de 100 Å.

La dispersion aqueuse est ajustée selon la méthode de l'exemple 1 afin d'obtenir un extrait sec de 20 %.

Exemple 10

20 g d'un ferrofluide dont le liquide vecteur est le toluène (ce qui correspond à 10 g de magnétite) sont introduits dans une solution constituée de 200 g de toluène et de 20 g d'un polyorganosiloxane présentant des insaturations éthyléniques obtenu par polycondensation d'oligomères polydiméthylsiloxanols en présence de 10 % en poids d'oligomères de tétraméthyltétravinylcyclotétrasiloxane, polymère dont les caractéristiques sont les suivantes :
$\overline{Mn}$ = 114.000 $\overline{Mw}$ = 222.000
Viscosité : 400.000 mPas à 25°C
Taux de Si-vinyle : 650 motifs par chaîne

Le mélange obtenu est homogénéisé en présence de 200 g d'eau et de 2 g de dodécylbenzènesulfonate de sodium dans une cuve à ultrasons (SONIFIER B 30). Après 10 minutes d'homogénéisation, on obtient une dispersion de microsphères magnétisables présentant une granulométrie moyenne de 0,75 micron. Après élimination du toluène, la granulométrie moyenne est de 0,5 micron.

La dispersion aqueuse est ajustée selon la méthode de l'exemple 1 afin d'obtenir un extrait sec de 20 %.

Les microsphères renferment 50 % en poids de particules magnétisables de taille de l'ordre de 100 Å.

Exemple 11

On répète l'opération décrite à l'exemple 10 en remplaçant le toluène par du cyclohexane comme liquide vecteur du ferrofluide et comme solvant du polyorganosiloxane et en remplaçant les 20 g de polyorganosiloxane de l'exemple 10 par 20 g d'un polyorganosiloxane présentant également des insaturations éthyléniques obtenu par polycondensation d'oligomères polydiméthylsiloxanols en présence de 5 % en poids (au lieu de 10 %) d'oligomères de tétraméthyltétravinylcyclotétrasiloxane dont les caractéristiques sont les suivantes :
$\overline{Mn}$ - 48.500 $\overline{Mw}$ 110.000
Viscosité : 9.500.000 mPas à 25°C
Taux de Si-vinyle : 140 motifs par chaîne

La granulométrie moyenne des microsphères magnétisables est de 0,8 micron avant élimination du cyclohexane et de 0,6 micron après élimination du cyclohexane.

La dispersion aqueuse est ajustée selon la méthode de l'exemple 1 afin d'obtenir un extrait sec de 13 %.

Les microsphères renferment 50 % en poids de particules magnétisables de taille de l'ordre de 100 Å.

Exemple 12

20 g d'un ferrofluide dont le liquide vecteur est le cyclohexane (ce qui correspond à 10 g de magnétite) sont introduits dans une solution constituée de 400 g de cyclohexane et du mélange des polymères suivants :
- 50 g du polydiméthylsiloxane α,ω hydroxylé de l'exemple 7 ($\overline{Mn}$ = 179.000, $\overline{Mw}$ = 535.000)
- 5 g d'une huile polydiméthylsiloxane époxydée de formule

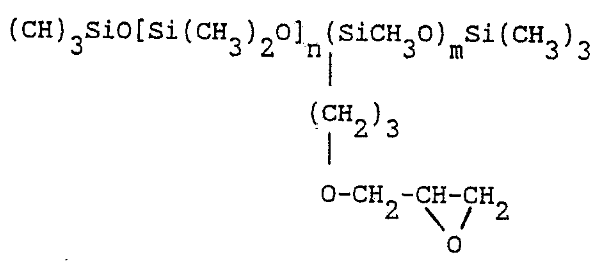

dont les caractéristiques sont les suivantes :
Mn = 5.000
Concentration en groupes époxydes : 174 meq/100 g d'huile (soit 8 motifs par chaîne d'huile époxydée).

La viscosité de l'ensemble des polyorganosiloxanes est de 6.000.000 mPas à 25°C.

Le mélange obtenu est homogénéisé en présence de 800 g d'eau et de 5,5 g de CEMULSOL ON 10-20 dans une cuve à ultra-sons (SONIFIER B 30). Après 10 minutes d'homogénéisation, on obtient une dispersion de microsphères magnétisables présentant à leur périphérie des groupes époxy. Ces microsphères présentent une granulométrie moyenne de 0,95 micron. Après élimination du cyclohexane, celle-ci est de 0,8 micron.

Les microsphères renferment 18 % en poids de particules magnétisables de taille de l'ordre de 100 Å.

La dispersion aqueuse est ajustée selon la méthode de l'exemple 1 afin d'obtenir un extrait sec de 36 %.

Exemple 13

8 g d'un ferrofluide dont le liquide vecteur est le cyclohexane (ce qui correspond à 4 g de magnétite) sont

introduits dans une solution constituée de 400 g de cyclohexane et du mélange des polymères suivants :
- 25 g du polydiméthylsiloxane α,ω hydroxylé de l'exemple 7 ($\overline{Mn}$ = 179.000, $\overline{Mw}$ = 535.000).
- 2 g d'une huile polydiméthylsiloxane de formule analogue à celle de l'huile époxydée de l'exemple 12, mais présentant les caractéristiques suivantes :
$\overline{Mn}$ = 1.250
Concentration en groupes époxydes : 498 meq/100 g d'huile (soit 15 motifs époxydés par chaîne d'huile époxydée).

La viscosité de l'ensemble des polyorganosiloxanes est de 6.000.000 mPas à 25°C.

Le mélange obtenu est ensuite traité comme à l'exemple 12.

La granulométrie moyenne des microsphères magnétisables est de 0,8 micron avant élimination du cyclohexane et de 0,7 micron après élimination du cyclohexane.

Les microsphères renferment 13,3 % en poids de particules magnétisables de taille de l'ordre de 100 Å.

La dispersion aqueuse est ajustée selon la méthode de l'exemple 1 afin d'obtenir un extrait sec de 36 %.

Exemple 14

On répète l'opération de l'exemple 13 en remplaçant les 2 g d'huile polydiméthylpolysiloxane de $\overline{Mn}$ = 1.250 et présentant une concentration en groupes époxydes de 498 meq/100 g d'huile par 2 g d'une huile polydiméthylpolysiloxane de formule analogue de $\overline{Mn}$ = 1.250 et présentant une concentration en groupes époxydes de 390 meq/100 g d'huile (soit 9 motifs époxydes par chaîne d'huile époxydée).

La viscosité de l'ensemble des polyorganosilanes est de 6.000.000 mPas à 25°C.

La granulométrie moyenne des microsphères magnétisables est de 0,9 micron avant élimination du cyclohexane et de 0,8 micron après élimination du cyclohexane.

Les microsphères renferment 13,3 % en poids de particules magnétisables de l'ordre de 100 Å.

La dispersion aqueuse est ajustée selon la méthode de l'exemple 1 afin d'obtenir un extrait sec de 15 %.

**Revendications**

1. Microsphères magnétisables caractérisées en ce qu'elles sont constituées
- d'une matrice à base d'au moins un organopolysiloxane de formule (I)

$R''R'RSiO(SiR_2O)_n(SiRR''O)_mSiRR'R''$ (I)

formule dans laquelle :
. les symboles R sont identiques ou différents et représentent un radical alkyle en $C_1$-$C_3$, phényle ou trifluoro-3,3,3 propyle ;
. les symboles R' représentent un groupement OH ou le symbole R ;
. le symbole R'' représente le symbole R, un radical vinyle ou un radical -R'''-X, où R''' représente un radical organique bivalent et X un groupement réactif et/ou ionogène ;
. au moins 50 % des radicaux représentés par le symbole R sont des radicaux méthyles ;
. les symboles n et m représentent des nombres entiers ou fractionnés ayant une valeur suffisante pour assurer une viscoité du polymère de 20 à 10.000.000 mPas à 25°C et un nombre de motifs vinyliques réactifs et/ou ionogènes éventuels par molécule d'organopolysiloxane allant de 1 à 1.000 ;
- et, encapsulées dans ladite matrice, de particules magnétisables présentant une taille généralement inférieure à 300 Å.

2. Microsphères magnétisables selon la revendication 1 caractérisées en ce que le radical bivalent R''' est un radical alkylène linéaire ou ramifié en $C_1$-$C_{18}$, éventuellement prolongé par 1 à 5 groupes bivalents éthylène amine, par 1 à 50 groupes bivalents oxyde d'alkylène en $C_1$-$C_3$ ou par un groupe

$-O-CH_2-\underset{OH}{\underset{|}{CH}}-CH_2-$

; un radical polyoxyalkylène contenant de 1 à 50 chaînons oxyalkylène en $C_1$-$C_3$.

3. Microsphères magnétisables selon la revendication 1 ou 2 caractérisées en ce que le symbole X est une fonction époxy, hydroxy, carboxy, aldéhyde, ester, acétoester, mercapto, mercaptoester, mercaptoalcoxy, amino, alkylamino, dialkylamino, trialkylamino, ammonium quaternaire, aminoalcool, amido, hydrazide, hydrazino, halogénoalkyle en $C_1$-$C_3$, cyano, cyanato,

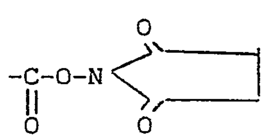

,

sulfonyle, halogénobenzoyle.

4. Microsphères magnétisables selon l'une quelconque des revendications précédentes caractérisées en ce que les particules magnétisables sont constituées de magnétite, magnémite, dioxyde de chrome, de ferrites telles que les ferrites de manganèse, nickel, manganèse-zinc, d'alliages de cobalt, nickel, gadolinium, samarium-cobalt.

5. Microsphères magnétisables selon la revendication 4 caractérisées en ce que les particules magnétisables sont constituées de magnétite.

6. Microsphères magnétisables selon l'une quelconque des revendications précédentes caractérisées en ce que leur diamètre va de 0,05 à 3 microns.

7. Microsphères magnétisables selon l'une quelconque des revendications précédentes caractérisées en ce qu'elles contiennent de 0,5 à 50 % en poids de particules magnétisables par rapport à la matrice.

8. Dispersions aqueuses de microsphères magnétisables caractérisées en ce qu'elles contiennent de 10 à 70 % de leur poids de microsphères magnétisables faisant l'objet de l'une quelconque des revendications précédentes.

9. Procédé de préparation de microsphères magnétisables comprenant les étapes consistant :

1) à introduire dans un milieu aqueux contenant un tensio-actif un mélange

. d'une solution d'au moins un organopolysiloxane de formule I définie à la revendication 1 dans un solvant organique de point d'ébullition inférieur à 100°C ou susceptible de former avec l'eau un azéotrope de point d'ébullition inférieur à 100°C

. et d'un fluide magnétique constitué de particules magnéti sables de taille généralement inférieure à 300 Å en suspension dans un liquide vecteur organique solvant dudit organopolysiloxane et de point d'ébullition inférieur à 100°C ou susceptible de former avec l'eau un azéotrope de point d'ébullition inférieur à 100°C et de préférence inférieur à 95°C.

2) à homogénéiser le milieu obtenu

3) à éliminer par distillation le solvant organique et le liquide vecteur organique

4) et à éliminer l'eau

10. Procédé de préparation de microsphères magnétisables en dispersion dans l'eau comprenant les étapes consistant :

1) à introduire dans un milieu aqueux contenant un tensio-actif un mélange

. d'une solution d'au moins un organopolysiloxane de formule I définie à la revendication 1 dans un solvant organique de point d'ébullition inférieur à 100°C ou susceptible de former avec l'eau un azéotrope de point d'ébullition inférieur à 100°C

. et d'un fluide magnétique constitué de particules magnétisables de taille généralement inférieure à 300 Å en suspension dans un liquide vecteur organique solvant dudit organopolysiloxane et de point d'ébullition inférieur à 100°C ou susceptible de former avec l'eau un azéotrope de point d'ébullition inférieur à 100°C et de préférence inférieur à 95°C.

2) à homogénéiser le milieu obtenu

3) à éliminer par distillation le solvant organique et le liquide vecteur organique

4) et à ajuster la concentration en microsphères magnétisables à une valeur allant de 10 à 60 % en poids par élimination partielle de l'eau ou par élimination totale de l'eau suivie d'une addition d'eau jusqu'à obtenir ladite concentration.

11. Procédé selon la revendication 9 ou 10 caractérisé en ce que le milieu aqueux contient de 0,5 à 15 % en poids de tensio-actif et est mis en oeuvre suivant une quantité correspondant à 0,5 à 60 % en poids de tensio-actif par rapport au poids d'organopolysiloxane(s) de formule I ;

12. Procédé selon l'une quelconque des revendications 9 à 11 caractérisé en ce que dans la formule I le radical bivalent R''' est un radical alkylène linéaire ou ramifié en $C_1$-$C_{18}$, éventuellement prolongé par 1 à 5 groupes bivalents éthylène amine, par 1 à 50 groupes bivalents oxyde d'alkylène en $C_1$-$C_3$ ou par un groupe $-O-CH_2-\underset{OH}{\underset{|}{CH}}-CH_2-$

; un radical polyoxyalkylène contenant de 1 à 50 chaînons oxyalkylène en $C_1$-$C_3$.

13. Procédé selon l'une quelconque des revendications 9 à 12 caractérisé en ce que dans la formule I le symbole X est une fonction époxy, hydroxy, carboxy, aldéhyde, ester, acétoester, mercapto, mercaptoester, mercaptoalcoxy, amino, alkylamino, dialkylamino, trialkylamino, ammonium quaternaire, aminoalcool, amido, hydrazide, hydrazino, halogénoalkyle en $C_1$-$C_3$, cyano, cyanato,

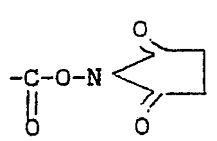

sulfate, sulfonyle, halogénobenzoyle.

14. Procédé selon l'une quelconque des revendications 9 à 13 caractérisé en ce que les particules magnétisables contenues dans le fluide magnétique sont constituées de magnétite, magnémite, dioxyde de chrome, de ferrites telles que les ferrites de manganèse, nickel, manganèse-zinc, d'alliages de cobalt,

nickel, gadolinium, samarium-cobalt.

15. Procédé selon la revendication 14 caractérisé en ce que les particules magnétisables contenues dans le fluide magnétique sont constituées de magnétite.

16. Procédé selon l'une quelconque des revendications 9 à 15 caractérisé en ce que le fluide magnétique contient de 20 à 60 % en poids de particules magnétisables.

17. Procédé selon l'une quelconque des revendications 9 à 16 caractérisé en ce que le poids de particules magnétisables du fluide magnétique correspond à 0,5 à 50 % du poids de polyorganosiloxane.

18. Procédé selon l'une quelconque des revendications 9 à 17 caractérisé en ce que le solvant de l'organopolysiloxane et le fluide vecteur du fluide magnétique sont semblables ou différents et sont du cyclohexane, du chlorure de méthylène, du benzène, de l'hexane, de l'octane, du toluène, du tétrachlorure de carbone.

19. Procédé selon l'une quelconque des revendications 9 à 18 caractérisé en ce que l'opération d'homogénéisation est réalisée jusqu'à obtenir des gouttelettes de 0,065 à 3,2 microns.

20. Procédé selon l'une quelconque des revendications 9 à 19 caractérisé en ce que l'élimination de l'eau est réalisée par aimantation.

21. Utilisation des microsphères magnétisables et dispersions de microsphères magnétisables faisant l'objet des revendications 1 à 8

Office européen des brevets

# RAPPORT DE RECHERCHE EUROPEENNE

Numero de la demande

EP 88 40 1740

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | CH-A- 397 735 (IBM)<br>* Page 1, lignes 30-32; page 2, lignes 15-39; page 3, lignes 64-81 *<br>--- | 1,4-6,9,10,14 | A 61 K 9/50<br>C 08 L 83/04<br>C 08 K 3/08<br>B 01 J 13/02 |
| A | GB-A-1 141 186 (THE NATIONAL CASH REGISTER CO.)<br>* Page 3, lignes 65-84 *<br>--- | 1,9,10 | |
| A | GB-A-2 045 790 (TORAY SILICONE CO.)<br>* Page 1, lignes 22-63; page 2, lignes 14-61; page 3, lignes 1-18 *<br>--- | 1 | |
| A | DE-A-3 002 477 (WALKER SCIENTIFIC INC.)<br>* Page 6, paragraphe 3 *<br>----- | 1,20 | |
| | | | **DOMAINES TECHNIQUES RECHERCHES (Int. Cl.4)** |
| | | | A 61 K<br>B 01 J<br>C 09 D<br>C 08 K<br>C 08 J<br>C 08 L |

Le présent rapport a été établi pour toutes les revendications

| Lieu de la recherche | Date d'achèvement de la recherche | Examinateur |
|---|---|---|
| LA HAYE | 06-10-1988 | KERRES P.M.G. |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)